# EUROPEAN PATENT APPLICATION

(11) **EP 2 305 222 A1**
(43) Date of publication of application: **06.04.2011**
(21) Application number: 10156391.4
(22) Date of filing: 04.04.2007
(51) Int. Cl.: A61K 31/10, A61K 31/355, A61K 31/375, A23K 1/16, A23K 1/18, A61P 3/02

(54) **Compositions and methods for enhancing the antioxidant status of animals**

(30) Priority: 04.04.2006 US 789021 P
(62) Divisional of application: 07754933.5
(71) Applicant: Hill's Pet Nutrition, Inc., Topeka, KS 66603 (US)
(72) Inventor: Yamka, Ryan, Topeka, KS 66610 (US); Friesen, Kim, Gene, Topeka, KS 66614 (US)
(74) Representative: Daniels, Jeffrey Nicholas

(57) **Abstract**

Compositions suitable for enhancing the antioxidant status of animals containing methionine, taurine, vitamin C, and vitamin E and methods for preparing and using such compositions to enhance the antioxidant status of animals are disclosed.

## Description

### BACKGROUND OF THE INVENTION

This invention relates generally to compositions for enhancing the antioxidant status of animals. The invention also relates generally to methods for preparing and using such compositions, articles of manufacture comprising such compositions, and means for communicating information about such compositions, methods, and articles of manufacture.

Living cells continuously produce free radicals during their normal functions. Free radicals are highly chemically reactive atoms, molecules, or molecular fragments with a free or unpaired electron such as, for example, reactive oxygen species and reactive nitrogen species. Free radicals are capable of reacting irreversibly with many biological molecules, thus causing progressive deterioration of the biological system. Free radicals are normally neutralized by the body's production of antioxidant enzymes and by antioxidants absorbed from nutrients. An antioxidant is any substance that reduces oxidative damage, for example, the damage caused by free radicals.

Antioxidant status is the balance between pro-oxidants and the antioxidant system. A serious imbalance favoring oxidation is defined as oxidative stress. Oxidative stress may result from excessive production of free radicals. Oxidative stress may also result from the weakening of the antioxidant system due to lower intake of antioxidants, lower endogenous production of antioxidants, and/or increased utilization of antioxidants.

Oxidative stress may cause cell damage, thus contributing to aging and developing chronic diseases. Prevention and/or lowering of oxidative stress by enhancing an animal's antioxidant system (thereby enhancing the animal's antioxidant status) may be important for good health and disease prevention and treatment, particularly in the case of older animals. Thus, there is a need for compositions and methods for enhancing the antioxidant status of animals.

### SUMMARY OF THE INVENTION

It is an object of this invention to provide compositions suitable for enhancing the antioxidant status of animals.

It is another object to provide methods for enhancing the antioxidant status of animals.

It is another object to provide articles of manufacture comprising a composition of the invention or two or more ingredients that, when combined together and, optionally, with additional ingredients, yield a composition of the invention that is suitable for enhancing the antioxidant status of animals.

It is yet another object to provide means for communicating information about the compositions, methods, and articles of manufacture of the invention.

Additional objects, features, and advantages of the present invention will be readily apparent to those skilled in the art.

### DETAILED DESCRIPTION OF THE INVENTION

Oxidative stress may cause cell damage, thus contributing to aging and developing chronic diseases. Prevention and/or lowering of oxidative stress by enhancing an animal's antioxidant status may be important for good health in general as well as for prevention and treatment of disease(s). Thus, in one aspect, this invention provides compositions suitable for enhancing the antioxidant status of an animal, thus improving the animal's health and/or enhancing the animal's quality of life. The compositions can help lower the amount of pro-oxidants (*e*.*g*., free radicals) in the animal's blood. The compositions can also improve the oxygen radical absorbance capacity ("ORAC") of the animal's blood, increase the amount of antioxidants in the animal's blood, lower oxidative stress-related cell damage, and/or lower the damage to the animal's deoxyribonucleic acid ("DNA").

The compositions are suitable for growing, adult, senior, and geriatric animals. A growing animal is one that has not reached adult size. An adult animal is one of any age after completion of juvenile growth and development. For example, for cats and dogs this typically means an age of about 1 year through the remainder of their life. A senior animal is one of an age having an increased risk for age-related disease which may but need not have obvious physical or behavioral characteristics of aging. For example, typically, but also depending on breed, a senior dog is one of about 7 through about 9 years of age, a senior large breed dog is one of about 5 years of age and beyond, and a senior cat is one of about 7 through about 11 years of age. A geriatric animal is one showing outward signs of aging, typically, for example, a dog of about 10 years of age and beyond, a large breed dog of about 5 years of age and beyond, or a cat of about 12 years of age and beyond.

The compositions of the invention are suitable for human and non-human animals, including non-human mammals such as non-human primates (*e*.*g*., monkeys, chimpanzees, etc.), companion and working animals (*e*.*g*., horses, etc.), farm animals (*e*.*g*., goats, sheep, pigs, cattle, etc.), and wild and zoo animals (*e*.*g*., wolves, bears, deer, etc.). They also are suitable for use with non-mammalian animals, such as companion, farm, zoo, and wild birds, (including, for example, song birds, parrots, ducks, geese, chickens, turkeys, ostriches, etc.).

In some embodiments, the compositions of the invention are suitable for animals that are members of the order *Carnivora.* In some such embodiments, the animal is a canine, and in others is a feline. In some such embodiments, the canine is a dog, and the feline is a cat.

In some embodiments, the compositions are suitable for companion animals. A companion animal can be, for example, an animal of any species kept as a pet. A companion animal can also be an animal from a variety of widely domesticated species, for example, dogs (*Canis familiaris*) and cats (*Felis domesticus*) regardless of whether or not it is kept solely as a pet. Thus, companion animals include working animals as well as pet animals.

In some embodiments, the compositions of the invention are suitable for animals whose life expectancy at birth is greater than about 1 year, for example, greater than about 3 or greater than about 5 years. Thus, in some embodiments, the compositions are suitable for non-murine animals (*i*.*e*., animals other than rodents of the family *Muridae*)*.*

The compositions of the invention comprise methionine, taurine, vitamin C, and vitamin E. Methionine, taurine, vitamin C, and vitamin E possess antioxidant properties.

Methionine is an essential sulfur-containing amino acid required in an animal's diet for balanced nutrition. Both pure methionine and methionine-comprising compositions are suitable for preparing compositions of the invention. For example, fruits, vegetables, and legumes (*e*.*g*., spinach, green peas, corn, navel and mandarin oranges, macadamia nuts, peanuts, pistachios, and.kidney beans) are rich in methionine.

In some embodiments, a composition of the invention comprises from about 0.9 to about 1.5% methionine. Unless otherwise stated, all percentages provided herein are weight percentages on a dry matter basis. On a "dry matter basis" means that an ingredient's concentration in a composition is measured after removing all moisture from the composition. In some such embodiments, the composition comprises from about 0.9 to about 1.1, about 1.2, about 1.3, about 1.4, or about 1.5% methionine. In other such embodiments, the compositions comprise from about 1.1 or about 1.2 to about 1.3, about 1.4, or about 1.5% methionine (*i*.*e*., the compositions comprise from about 1.1 to about 1.3, from about 1.1 to about 1.4, from about 1.1 to about 1.5, from about 1.2 to about 1.3, from about 1.2 to about 1.4, or from about 1.2 to about 1.5% methionine).

In some such embodiments, the composition comprises from about 0.9 to about 1.3 or from about 0.9 to about 1.1% methionine. In some embodiments, such composition is suitable for enhancing the antioxidant status of a canine. In some such embodiments, the canine is a dog. In some such embodiments, the canine is a senior canine (*e*.*g*., a senior dog); while in other such embodiments, the canine is a geriatric canine (*e*.*g*., a geriatric dog).

In other such embodiments, the composition comprises from about 1.1 to about 1.5 or from about 1.2 to about 1.4% methionine. In some embodiments, such composition is suitable for enhancing the antioxidant status of a feline. In some such embodiments, the feline is a cat. In some such embodiments, the feline is a senior feline (*e*.*g*., a senior cat); while in other such embodiments, the canine is a geriatric feline (*e*.*g*., a geriatric cat).

Taurine is a sulfur-containing amino acid that is required in an animal's diet for balanced nutrition. Taurine is an essential amino acid for some animals (*e*.*g*., cats), and is possibly conditionally essential for other animals (*e*.*g*., dogs). Both pure taurine and taurine-comprising compositions are suitable for preparing compositions of the invention. For example, shellfish and organ meats (*e*.*g*., liver) are rich in taurine.

In some embodiments, a composition of the invention comprises from about 1200 to about 5000 parts per million ("ppm") taurine. 1ppm taurine equals 1mg taurine per 1kg composition after removing all moisture from the composition. In some such embodiments, the composition comprises from about 1200 to about 1300, about 1400, about 1500, about 1600, about 1700, about 1800, about 1900, or about 2000ppm taurine. In other such embodiments, the composition comprises from about 2000, about 2200, or about 2400 to about 3000, about 3500, about 4000, about 4500, or about 5000ppm taurine.

In some such embodiments, the composition comprises from about 1200 to about 1500 or from about 1200 to about 1400ppm taurine. In some embodiments, such composition is suitable for enhancing the antioxidant status of a canine. In some such embodiments, the canine is a dog. In some such embodiments, the canine is a senior canine (*e*.*g*., a senior dog); while in other such embodiments, the canine is a geriatric canine (*e*.*g*., a geriatric dog).

In other such embodiments, the composition comprises from about 2000 to about 5000 or from about 2200 to about 4000ppm taurine. In some embodiments, such composition is suitable for enhancing the antioxidant status of a feline. In some such embodiments, the feline is a cat. In some such embodiments, the feline is a senior feline (*e*.*g*., a senior cat); while in other such embodiments, the canine is a geriatric feline (*e*.*g*., a geriatric cat).

Vitamin C (*i*.*e*., L-ascorbic acid) primarily functions in the body as an antioxidant and a free radical scavenger. Pure vitamin C or derivatives thereof (e.g., salts such as, for example, sodium, calcium, zinc, and ferrous salts; phosphates; esters such as, for example, stearates and palmitates) as well as compositions comprising vitamin C or the derivatives thereof are suitable for preparing compositions of the invention. Fruits and vegetables as well as organ meats are rich in vitamin C.

In some embodiments, a composition of the invention comprises from about 120 to about 450 ppm vitamin C. In some such embodiments, the composition comprises from about 120, about 130, or about 140 to about 200, about 300, or about 400ppm vitamin C. In other such embodiments, the composition comprises from about 120, about 130, about 140, or about 150 to about 160, about 170, about 180, about 190, about 200, about 250, about 300, about 350, about 400, or about 450ppm vitamin C.

Vitamin E functions as an antioxidant *in vivo* and *in vitro.* It is believed that vitamin E works in conjunction with glutathione peroxidase to protect cells against the adverse effects of reactive oxygen and other free radicals that initiate the oxidation of polyunsaturated membrane phospholipids. Vitamin E is synthesized only by plants. There are eight isomeric forms of vitamin E: α-, β-, γ-, and δ-tocopherols and α-, β-, γ-, and δ-tocotrienols, with α-tocopherol being the most active biopotent form. Pure vitamin E or derivatives thereof as well as compositions comprising vitamin E or the derivatives thereof are suitable for preparing compositions of the invention. Vegetable oils, seeds, and cereal grains are rich in vitamin E, with tocopherol concentrations being highest in green leaves.

In some embodiments, a composition of the invention comprises from about 700 to about 2000IU/kg vitamin E (*i*.*e*., the composition comprises from about 700 to about 2000 international units of vitamin E per kg of the composition after removing all moisture from the composition). In some such embodiments, the composition comprises from about 800, about 1000, or about 1100 to about 1200, about 1400, about 1500, about 1600, about 1700, about 1800, about 1900, or about 2000IU/kg vitamin E. In other such embodiments, the composition comprises from about 1100, about 1150, about 1200, about 1250, about 1300, about 1350, or about 1400 to about 1600, about 1700, about 1800, about 1900, or about 2000IU/kg vitamin E.

In some such embodiments, the composition comprises from about 700 to about 2000 or from about 900 to about 2000IU/kg vitamin E. In some embodiments, such composition is suitable for enhancing the antioxidant status of a canine. In some such embodiments, the canine is a dog. In some such embodiments, the canine is a senior canine (*e*.*g*., a senior dog); while in other such embodiments, the canine is a geriatric canine (*e*.*g*., a geriatric dog).

In other such embodiments, the composition comprises from about 1100 to about 2000 or from about 700 to about 2000IU/kg vitamin E. In some embodiments, such composition is suitable for enhancing the antioxidant status of a feline. In some such embodiments, the feline is a cat. In some such embodiments, the feline is a senior feline (*e*.*g*., a senior cat); while in other such embodiments, the canine is a geriatric feline (*e*.*g*., a geriatric cat).

As discussed above, a composition of this invention can comprise methionine, taurine, vitamin C, and vitamin E in any combination of the amounts of those antioxidants discussed above. For example, in some embodiments, the composition comprises from about 0.9 to about 1.5% methionine, from about 1200 to about 5000 ppm taurine, from about 120 to about 450 ppm vitamin C, and from about 700 to about 2000IU/kg vitamin E.

In some such embodiments, the composition comprises from about 0.9 to about 1.3% methionine, from about 1200 to about 1500 ppm taurine, from about 120 to about 250 ppm vitamin C, and from about 900 to about 2000IU/kg vitamin E. In some such embodiments, the composition comprises from about 0.9 to about 1.1% methionine, from about 1200 to about 1400 ppm taurine, from about 120 to about 250 ppm vitamin C, and from about 900 to about 2000IU/kg vitamin E.

In other such embodiments, the composition comprises from about 1.1 to about 1.5% methionine, from about 2000 to about 5000 ppm taurine, from about 130 to about 250 ppm vitamin C, and from about 700 to about 2000IU/kg vitamin E. In some such embodiments, the composition comprises from about 1.2 to about 1.4% methionine, from about 2200 to about 4000 ppm taurine, from about 130 to about 250 ppm vitamin C, and from about 700 to about 2000IU/kg vitamin E.

In yet other such embodiments, the composition comprises from about 0.9 to about 1.1, about 1.2, about 1.3, about 1.4, or about 1.5% methionine; from about 1200 to about 1300, about 1400, about 1500, about 1600, about 1700, about 1800, about 1900, or about 2000ppm taurine; from about 120, about 130, or about 140 to about 200, about 300, or about 400ppm vitamin C; and from about 800, about 1000, or about 1100 to about 1200, about 1400, about 1500, about 1600, about 1700, about 1800, about 1900, or about 2000IU/kg vitamin E.

In further such embodiments, the composition comprises from about 1.1 or about 1.2 to about 1.3, about 1.4, or about 1.5% methionine; from about 2000, about 2200, or about 2400 to about 3000, about 3500, about 4000, about 4500, or about 5000ppm taurine; from about 120, about 130, about 140, or about 150 to about 160, about 170, about 180, about 190, about 200, about 250, about 300, about 350, about 400, or about 450ppm vitamin C; and from about 1100, about 1150, about 1200, about 1250, about 1300, about 1350, or about 1400 to about 1600, about 1700, about 1800, about 1900, or about 2000IU/kg vitamin E.

In some embodiments, a composition of the invention further comprises one or more antioxidants or antioxidant-comprising compositions in addition to the methionine, taurine, vitamin C, and vitamin E. Such antioxidants and antioxidant-comprising compositions include, for example, selenium, ubiquinone, lutein, glutathione, lipoic acid, spinach pomace, tomato pomace, grape pomace, carnitine, and citrus pulp.

In some embodiments, a composition of the invention, when fed to an animal, can increase the amount (or concentration) of one or more of methionine, taurine, vitamin C, vitamin E, and glutathione peroxidase in the animal's blood compared to the amount of the substance(s) before feeding the composition of the invention. In some embodiments, a composition of the invention, when fed to an animal, can increase the animal's ORAC (as measured by, for example, an ORAC increase in the animal's blood). In some embodiments, a composition of the invention can be fed to an animal for, for example, at least about three weeks, at least about four weeks, at least about eight weeks, at least about sixteen weeks, or at least about twenty-four weeks before an increase in the amount of, for example, one or more of methionine, taurine, vitamin C, vitamin E, glutathione peroxidase, and ORAC in the animal's blood is observed. One skilled in the art would understand that either a single composition of the invention can be fed to the animal for the entire period of time, or, alternatively, different compositions of the invention can be fed to the animal for varying time periods. One skilled in the art can also determine the amount of methionine, taurine, vitamin C, vitamin D, glutathione peroxidase, and ORAC in an animal's blood.

In some such embodiments, upon feeding a composition of the invention to a canine, the canine's blood comprises from about 300 to about 500µmol /L taurine. Taurine concentrations can be determined by methods known to skilled artisans. In some such embodiments, upon feeding a composition to a canine, the canine's blood comprises from about 300 to about 350µmol /L taurine.
In some embodiments, the canine is a dog. The canine can be, for example, a senior canine (*e*.*g*., a senior dog). It can also be a geriatric canine (*e*.*g*., a geriatric dog).

In other such embodiments, upon feeding a composition of the invention to a feline, the feline's blood comprises from about 460 to about 1000µmol /L taurine. In some such embodiments, upon feeding a composition to a feline, the feline's blood comprises from about 460 to about 500µmol /L taurine. In some embodiments, the feline is a cat. The feline can be, for example, a senior feline (*e*.*g*., a senior cat). It can also be a geriatric feline (*e*.*g*., a geriatric cat).

In some embodiments, a composition of the present invention comprises a food composition (*i*.*e*., the compositions comprise one or more food compositions). In some such embodiments, the composition is a food (*e*.*g*., pet food). Such food can be a dry, semi-moist, or moist food. In some embodiments, the food composition comprises a dry food. In some embodiments, the food composition comprises a semi-moist food. In some embodiments, the food composition comprises a moist food. In some embodiments, the food composition comprises a treat, snack, supplement, or partially or fully edible toy. In some embodiments, the composition comprises a mixture of one or more foods. In some embodiments, the composition meets the ordinary nutritional requirements of the animal that it is being fed to. In some embodiments, a composition of the invention meets AAFCO's minimum nutrient level requirements for growth, reproduction, and/or maintenance. In other embodiments, the composition comprises ingredients (*e*.*g*., vitamin E) in amounts higher than the AAFCO's maximum nutrient level requirements.

In another aspect, the invention provides methods for preparing compositions of the invention. Such compositions can be prepared by mixing various ingredients (including food compositions). Compositions of this invention can also be prepared by the methods discussed in, for example, Small Animal Nutrition, pages 127-146 (2000).

In another aspect, the invention provides a method for enhancing the antioxidant status of an animal. In another aspect, the invention provides a method for improving an animal's health. In another aspect, the invention provides a method for enhancing an animal's quality of life. In another aspect, the invention provides a method for lowering the amount of pro-oxidants in an animal's blood. In another aspect, the invention provides a method for improving an animal's ORAC. In another aspect, the invention provides a method for increasing the amount of antioxidants in an animal's blood. In another aspect, the invention provides a method for lowering the damage to an animal's DNA. The above methods comprise feeding the animal a composition of the invention discussed above (*i*.*e*., the above methods comprise feeding the animal a composition that comprises methionine, taurine, vitamin C, vitamin E, and, optionally, other antioxidants). One skilled in the art would understand that either a single composition of the invention can be fed to the animal, or, alternatively, different compositions of the invention can be fed to the animal for varying time intervals.

In some embodiments, the methods comprise feeding the animal a composition that comprises from about 0.9 to about 1.5% methionine, from about 1200 to about 5000 ppm taurine, from about 120 to about 450 ppm vitamin C, and from about 700 to about 2000IU/kg vitamin E.

In some embodiments, the methods comprise feeding the animal a composition that comprises from about 0.9 to about 1.3% methionine, from about 1200 to about 1500 ppm taurine, from about 120 to about 250 ppm vitamin C, and from about 900 to about 2000IU/kg vitamin E. In some such embodiments, the methods comprise feeding the animal a composition that comprises from about 0.9 to about 1.1 % methionine, from about 1200 to about 1400 ppm taurine, from about 120 to about 250 ppm vitamin C, and from about 900 to about 2000IU/kg vitamin E.

In some embodiments, the methods comprise feeding the animal a composition that comprises from about 1.1 to about 1.5% methionine, from about 2000 to about 5000 ppm taurine, from about 130 to about 250 ppm vitamin C, and from about 700 to about 2000IU/kg vitamin E. In some such embodiments, the methods comprise feeding the animal a composition that comprises from about 1.2 to about 1.4% methionine, from about 2200 to about 4000 ppm taurine, from about 130 to about 250 ppm vitamin C, and from about 700 to about 2000IU/kg vitamin E.

In some embodiments, the methods comprise feeding the animal a composition that comprises from about 0.9 to about 1.1, about 1.2, about 1.3, about 1.4, or about 1.5% methionine; from about 1200 to about 1300, about 1400, about 1500, about 1600, about 1700, about 1800, about 1900, or about 2000ppm taurine; from about 120, about 130, or about 140 to about 200, about 300, or about 400ppm vitamin C; and from about 800, about 1000, or about 1100 to about 1200, about 1400, about 1500, about 1600, about 1700, about 1800, about 1900, or about 2000IU/kg vitamin E.

In some embodiments, the methods comprise feeding the animal a composition that comprises from about 1.1 or about 1.2 to about 1.3, about 1.4, or about 1.5% methionine; from about 2000, about 2200, or about 2400 to about 3000, about 3500, about 4000, about 4500, or about 5000ppm taurine; from about 120, about 130, about 140, or about 150 to about 160, about 170, about 180, about 190, about 200, about 250, about 300, about 350, about 400, or about 450ppm vitamin C; and from about 1100, about 1150, about 1200, about 1250, about 1300, about 1350, or about 1400 to about 1600, about 1700, about 1800, about 1900, or about 2000IU/kg vitamin E.

If an animal suffers from one or more diseases, it may be desired to feed the animal a composition of the invention in conjunction with the administration of one or more agents that can help promote the animal's health. Thus, in some embodiments, the above methods further comprise administering to the animal one or more agents for promoting the health or wellness of the animal.

Health of an animal refers to the absence of disease or infirmity. Wellness refers to the complete physical, mental, and social wellbeing of the animal, not merely the absence of infirmity. The term "in conjunction" means that an agent is administered to the animal either together with a composition of the invention or separately from the composition at the same or different frequency via the same or different administration route and either at about the same time as the composition or periodically. "Administering" means that the agent is introduced in a suitable dosage form into the animal by a suitable administration route, for example, orally, topically, or parenterally. "About at the same time" generally means that an agent is administered when a composition of this invention is fed to the animal or within about 72 hours of feeding the composition to the animal. "Periodically" generally means that an agent is administered to an animal following a dosage schedule suitable for administering the agent while a composition of this invention is fed to the animal routinely as appropriate for that animal. Thus, the term "in conjunction" specifically includes situations when an agent is administered to an animal for a prescribed period of time while a composition of this invention is fed to the animal for a much longer period of time (e.g., for life). If more than one agent is administered, the dosage form and route of administration for each agent may vary. In addition, one composition of this invention may be substituted with another composition of this invention while a specific agent is administered to the animal.

An agent for promoting health or wellness can, for example, improve an animal's cognitive functions or the appearance and thickness of an animal's hair coat, or ameliorate or treat a disease that the animal suffers from.

In some embodiments, the agent for promoting health or wellness comprises one or more essential fatty acids such as, for example, omega-6 or omega-3 fatty acids. Omega-6 essential fatty acids include, for example, linoleic acid and arachidonic acid. Omega-3 essential fatty acids include, for example, alpha-linolenic acid, eicosapentaenoic acid, and docosahexaenoic acid. Essential fatty acids serve as substrates that may be metabolized to form important, biologically active compounds. Arachidonic acid, gamma-linolenic acid, and eicosapentaenoic acid act as precursors for the synthesis of eicosanoids, an important group of immunoregulatory molecules that function as local hormones and mediators of inflammation. Linoleic acid incorporates into the ceramides of the epidermal cornified envelope, which serves an essential barrier function to prevent loss of water and other nutrients from the skin. Essential fatty acid may be used in the form of various derivatives, for example, salts of inorganic and organic acids, phospolipid esters, ethers, and sterol derivatives. Linoleic and linolenic acids can be used as, for example, phosphatidal choline esters, phosphatidal ether, and sipolsterol ester.

In another aspect, this invention provides a use of a composition of this invention to prepare a medicament for one or more of: enhancing the antioxidant status of an animal, improving an animal's health, enhancing an animal's quality of life, lowering the amount of pro-oxidants in an animal's blood, improving an animal's ORAC, increasing the amount of antioxidants in an animal's blood, and lowering the damage to an animal's DNA. As discussed above, that composition comprises methionine, taurine, vitamin C, vitamin E, and, optionally, other antioxidants. In some such embodiments, the composition comprises from about 0.9 to about 1.5% methionine, from about 1200 to about 5000 ppm taurine, from about 120 to about 450 ppm vitamin C, and from about 700 to about 2000IU/kg vitamin E.

In another aspect, this invention provides for a use of methionine, taurine, vitamin C, vitamin E, and, optionally, other antioxidants to prepare a composition to (1) enhance the antioxidant status of an animal, (2) improve an animal's health, (3) enhance an animal's quality of life, (4) lower the amount of pro-oxidants in an animal's blood, (5) improve an animal's ORAC, (6) increase the amount of antioxidants in an animal's blood, and/or (7) lower the damage to an animal's DNA. In some embodiments, the composition comprises from about 0.9 to about 1.5% methionine, from about 1200 to about 5000 ppm taurine, from about 120 to about 450 ppm vitamin C, and from about 700 to about 2000IU/kg vitamin E.

In a further aspect, this invention provides an article of manufacture, for example, a kit comprising a composition of the invention as discussed above. The kit is suitable for one or more of: enhancing the antioxidant status of an animal, improving an animal's health, enhancing an animal's quality of life, lowering the amount of pro-oxidants in an animal's blood, improving an animal's ORAC, increasing the amount of antioxidants in an animal's blood, and lowering the damage to an animal's DNA. In some embodiments, the kit further comprises an agent for promoting the health or wellness of the animal (*i*.*e*., one or more agents for promoting health or wellness). In some embodiments, the kit further comprises instructions for one or more of (1) feeding the composition to the animal, (2) administering to the animal an agent for promoting health or wellness in conjunction with feeding the animal the composition, (3) enhancing the antioxidant status of the animal by feeding the animal the composition, and, optionally, administering to the animal an agent for promoting health or wellness in conjunction with feeding the animal the composition, (4) improving the animal's health by feeding the animal the composition, and, optionally, administering to the animal an agent for promoting health or wellness in conjunction with feeding the animal the composition, (5) enhancing the animal's quality of life by feeding the animal the composition, and, optionally, administering to the animal an agent for promoting health or wellness in conjunction with feeding the animal the composition, (6) lowering the amount of pro-oxidants in the animal's blood by feeding the animal the composition, and, optionally, administering to the animal an agent for promoting health or wellness in conjunction with feeding the animal the composition, (7) improving the animal's ORAC by feeding the animal the composition, and, optionally, administering to the animal an agent for promoting health or wellness in conjunction with feeding the animal the composition, (8) increasing the amount of antioxidants in the animal's blood by feeding the animal the composition, and, optionally, administering to the animal an agent for promoting health or wellness in conjunction with feeding the animal the composition, and (9) lowering the damage to the animal's DNA by feeding the animal the composition and, optionally, administering to the animal an agent for promoting health or wellness in conjunction with feeding the animal the composition.

In some embodiments of the invention, the kit comprises two or more ingredients that, when combined together and optionally with additional ingredients that are or are not a part of the kit, yield a composition of the invention. If such additional ingredients are to be used, the kit provides instructions about these ingredients. The ingredients can, for example, be pure antioxidants or derivatives thereof (*e*.*g*., pure methionine, taurine, vitamin C, and/or vitamin E or pure derivatives of, for example, vitamins C and E) as well mixtures of these antioxidants. The antioxidant mixtures may comprise additional antioxidants as well. The kit ingredients can also be compositions or raw materials containing methionine, taurine, vitamin C, and/or vitamin E, and, optionally, other antioxidants. And the ingredients can also be, for example, one or more foods and a mixture of antioxidants (*e.g*., a mineral mix) that, when mixed, yield a composition of the invention.

In some embodiments, the kit further comprises an agent for promoting health or wellness in an animal. In some embodiments, the kit further comprises instructions for one or more of (1) preparing a composition of the invention by combining the ingredients, (2) feeding the composition to the animal, (3) administering to the animal an agent for promoting health or wellness in conjunction with feeding the animal the composition, (4) enhancing the antioxidant status of the animal by feeding the animal the composition, and, optionally, administering to the animal an agent for promoting health or wellness in conjunction with feeding the animal the composition, (5) improving the animal's health by feeding the animal the composition, and, optionally, administering to the animal an agent for promoting health or wellness in conjunction with feeding the animal the composition, (6) enhancing the animal's quality of life by feeding the animal the composition, and, optionally, administering to the animal an agent for promoting health or wellness in conjunction with feeding the animal the composition, (7) lowering the amount of pro-oxidants in the animal's blood by feeding the animal the composition, and, optionally, administering to the animal an agent for promoting health or wellness in conjunction with feeding the animal the composition, (8) improving the animal's ORAC by feeding the animal the composition, and, optionally, administering to the animal an agent for promoting health or wellness in conjunction with feeding the animal the composition, (9) increasing the amount of antioxidants in the animal's blood by feeding the animal the composition, and, optionally, administering to the animal an agent for promoting health or wellness in conjunction with feeding the animal the composition, and (10) lowering the damage to the animal's DNA by feeding the animal the composition and, optionally, administering to the animal an agent for promoting health or wellness in conjunction with feeding the animal the composition.

In some embodiments, the kit comprises in separate containers in a single package or in separate containers in a virtual package, as appropriate, a composition of this invention or two or more ingredients, that, when combined together and optionally with additional ingredients that are or are not a part of the kit, yield a composition of the invention, and one or more of (1) an agent for promoting the health or wellness of the animal, (2) instructions for preparing a composition of the invention by combining the ingredients, (3) instructions for feeding the composition to the animal, (4) instructions for administering to the animal an agent for promoting health or wellness in conjunction with feeding the animal the composition, (5) instructions for enhancing the antioxidant status of the animal by feeding the animal the composition, and, optionally, administering to the animal an agent for promoting health or wellness in conjunction with feeding the animal the composition, (6) instructions for improving the animal's health by feeding the animal the composition, and, optionally, administering to the animal an agent for promoting health or wellness in conjunction with feeding the animal the composition, (7) instructions for enhancing the animal's quality of life by feeding the animal the composition, and, optionally, administering to the animal an agent for promoting health or wellness in conjunction with feeding the animal the composition, (8) instructions for lowering the amount ofpro-oxidants in the animal's blood by feeding the animal the composition, and, optionally, administering to the animal an agent for promoting health or wellness in conjunction with feeding the animal the composition, (9) instructions for improving the animal's ORAC by feeding the animal the composition, and, optionally, administering to the animal an agent for promoting health or wellness in conjunction with feeding the animal the composition, (10) instructions for increasing the amount of antioxidants in the animal's blood by feeding the animal the composition, and, optionally, administering to the animal an agent for promoting health or wellness in conjunction with feeding the animal the composition, and (11) instructions for lowering the damage to the animal's DNA by feeding the animal the composition and, optionally, administering to the animal an agent for promoting health or wellness in conjunction with feeding the animal the composition.

The term "single package" generally means that the components of a kit are physically associated in or with one or more containers and considered as a unit of manufacture, distribution, sale, or use. Containers include, for example, bags, boxes, bottles, shrink wrap packages, stapled or otherwise fixed components, and combinations thereof. A single package can be, for example, containers or individual food compositions physically associated such that they are considered a unit for manufacture, distribution, sale, or use. The term "virtual package" generally means that the components of a kit are associated by directions on one or more physical or virtual kit components instructing the user how to obtain additional components, e.g., in a bag containing one component and directions instructing the user to go to a website, contact a recorded message, view a visual message, or contact a caregiver to obtain instructions on how to use the kit. When the kit comprises a virtual package, the kit is limited to instructions in a virtual environment with one or more physical kit components.

In some embodiments, the kit is suitable for enhancing the antioxidant status of an animal and comprises in separate containers in a single package or in separate containers in a virtual package, as appropriate, a composition comprising from about 0.9 to about 1.5% methionine, from about 1200 to about 5000 ppm taurine, from about 120 to about 450 ppm vitamin C, and from about 700 to about 2000IU/kg vitamin E, or two or more ingredients that, when combined together and, optionally, with additional ingredients which are or are not a part of the kit, yield a composition comprising from about 28 to about 35% protein wherein the protein comprises from about 0.9 to about 1.5% methionine, from about 1200 to about 5000 ppm taurine, from about 120 to about 450 ppm vitamin C, and from about 700 to about 2000IU/kg vitamin E, and one or more of (1) an agent for promoting the health or wellness of the animal, (2) instructions for preparing the composition by combining the ingredients, (3) instructions for feeding the composition to the animal, (4) instructions for administering to the animal an agent for promoting health or wellness in conjunction with feeding the animal the composition, (5) instructions for enhancing the antioxidant status of the animal by feeding the animal the composition, and, (6) instructions for enhancing the antioxidant status of the animal by administering to the animal an agent for promoting health or wellness in conjunction with feeding the animal the composition.

In a further aspect, this invention provides a means for communicating information about or instructions for one or more of (1) feeding a composition of the invention to an animal, (2) administering to an animal an agent for promoting health or wellness in conjunction with feeding the animal a composition of the invention, (3) enhancing the antioxidant status of an animal by feeding the animal a composition of the invention, and, optionally, administering to the animal an agent for promoting health or wellness in conjunction with feeding the animal the composition, (4) improving an animal's health by feeding the animal a composition of the invention, and, optionally, administering to the animal an agent for promoting health or wellness in conjunction with feeding the animal the composition, (5) enhancing an animal's quality of life by feeding the animal a composition of the invention, and, optionally, administering to the animal an agent for promoting health or wellness in conjunction with feeding the animal the composition, (6) lowering the amount of pro-oxidants in an animal's blood by feeding the animal a composition of the invention, and, optionally, administering to the animal an agent for promoting health or wellness in conjunction with feeding the animal the composition, (7) improving an animal's ORAC by feeding the animal a composition of the invention, and, optionally, administering to the animal an agent for promoting health or wellness in conjunction with feeding the animal the composition, (8) increasing the amount of antioxidants in an animal's blood by feeding the animal the composition, and, optionally, administering to the animal an agent for promoting health or wellness in conjunction with feeding the animal the composition, and (8) lowering the damage to an animal's DNA by feeding the animal a composition of the invention and, optionally, administering to the animal an agent for promoting health or wellness in conjunction with feeding the animal the composition. The communication means comprise, for example, a document, digital storage media, optical storage media, audio presentation, or visual display containing the information or instructions. Preferably, the communication means is a displayed web site or a brochure, product label, package insert, advertisement, or visual display containing such information or instructions. Useful information or instructions include, for example, (1) information and instructions how to use a composition, method, or kit of the invention and (2) contact information for animal caregivers if they have a question about the invention and its uses.

In some embodiments, the present invention provides a means for communicating information about or instructions for one or more of (1) feeding an animal a composition comprising from about 0.9 to about 1.5% methionine, from about 1200 to about 5000 ppm taurine, from about 120 to about 450 ppm vitamin C, and from about 700 to about 2000IU/kg vitamin E, (2) administering to an animal an agent for promoting the health or wellness of the animal in conjunction with feeding the animal a composition comprising from about 0.9 to about 1.5% methionine, from about 1200 to about 5000 ppm taurine, from about 120 to about 450 ppm vitamin C, and from about 700 to about 2000IU/kg vitamin E, (3) enhancing the antioxidant status of an animal by feeding the animal a composition comprising from about 0.9 to about 1.5% methioriine, from about 1200 to about 5000 ppm taurine, from about 120 to about 450 ppm vitamin C, and from about 700 to about 2000IU/kg vitamin E, (4) enhancing the antioxidant status of an animal by administering to the animal an agent for promoting health or wellness in conjunction with feeding the animal a composition comprising from about 0.9 to about 1.5% methionine, from about 1200 to about 5000 ppm taurine, from about 120 to about 450 ppm vitamin C, and from about 700 to about 2000IU/kg vitamin E, and (5) using a kit of the invention comprising a composition that comprises from about 0.9 to about 1.5% methionine, from about 1200 to about 5000 ppm taurine, from about 120 to about 450 ppm vitamin C, and from about 700 to about 2000IU/kg vitamin E, or two or more ingredients that, when combined together and, optionally, with additional ingredients, yield a composition that comprises from about 0.9 to about 1.5% methionine, from about 1200 to about 5000 ppm taurine, from about 120 to about 450 ppm vitamin C, and from about 700 to about 2000IU/kg vitamin E. The means comprise a document, digital storage media, audio presentation, or visual display containing the information or instructions.

In a further aspect, the present invention provides for a use of a composition comprising from about 0.9 to about 1.5% methionine, from about 1200 to about 5000 ppm taurine; from about 120 to about 450 ppm vitamin C, and from about 700 to about 2000IU/kg vitamin E, all on a dry matter basis, to prepare a medicament. In another, the invention provides for the use of such composition to prepare a medicament for enhancing the antioxidant status of an animal. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

In a further aspect, the invention provides for the use of the compositions, kits and methods of the present invention to promote and/or improve an animal's health and/or wellness, enhance the antioxidant status of the animal, lower the amount of pro-oxidants in the animal's blood, improve the animal's ORAC, increase the amount of antioxidants in the anima'ls blood, and lower the damage to the animals' DNA.

As stated previously, unless otherwise stated, all percentages provided herein, including for the kit and means for communicating, are weight percentages on a dry matter basis.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any compositions, methods, articles of manufacture, or other means or materials similar or equivalent to those described herein can be used in the practice of the present invention, the preferred compositions, methods, articles of manufacture, or other means or materials are described herein.

All patents, patent applications, publications, and other references cited or referred to herein are incorporated herein by reference to the extent allowed by law. The discussion of those references is intended merely to summarize the assertions made therein. No admission is made that any such patents, patent applications, publications or references, or any portion thereof, is relevant prior art for the present invention and the right to challenge the accuracy and pertinence of such patents, patent applications, publications, and other references is specifically reserved.

### Examples

This invention can be further illustrated by the following examples, although it will be understood that the examples are included merely for purposes of illustration, and are not intended to limit the scope of the invention unless otherwise specifically indicated.

### Example 1

Example 1 illustrates the effect of the compositions of this invention on enhancing the antioxidant status, improving ORAC, increasing antioxidant amount in blood, and lowering DNA damage in dogs.

Forty healthy geriatric beagle dogs (ten years old or older) are fed a control food meeting AAFCO's minimum nutrient level requirements for maintenance for thirty days. After that, the dogs are split in four groups, ten dogs per group, and each group is fed one of dry foods A, B, C, or D for six months. The contents of foods A, B, C, and D are listed in Table 1.

**Table 1**

| Composition of Foods A, B, C, and D | | | | |
|---|---|---|---|---|
| Food Ingredients (DMB) | Food A | Food B | Food C | Food D |
| Crude Protein (%) | 20.10 | 27.65 | 27.76 | 29.39 |
| Fat (%) | 16.45 | 13.52 | 11.08 | 13.59 |
| Calcium (%) | 0.71 | 0.79 | 1.28 | 1.35 |
| Phosphorus (%) | 0.61 | 0.68 | 0.93 | 1.14 |
| Taurine (ppm) | 1400 | 1090 | <100 | 1600 |
| Methionine (%) | 1.00 | 0.49 | 0.51 | 0.66 |
| Vitamin E (IU/kg) | 1492 | 594 | 894 | 421 |
| Vitamin C (ppm) | 127 | 288 | 86 | 21 |

Blood samples are drawn after the dogs are fed Food A, B, C, or D for one month to determine the amounts of methionine, taurine, vitamin E, and glutathione peroxidase in the blood, the ORAC of the blood, and the ability of the cells to withstand oxidative stress by hydrogen peroxide. Results are presented in Tables 2 and 3.

**Table 2**

| Results from Blood Analysis at One Month | | | | |
|---|---|---|---|---|
| Metabolite | Food A | Food B | Food C | Food D |
| Glutathione Peroxidase (µg/10⁶) | 5.85 | 5.68 | 5.68 | 5.96 |
| Taurine (µmol/L) | 305.4 | 269.5 | 282.9 | 286.2 |
| ORAC (µM) | 4925 | 5048 | 4798 | 4803 |
| Vitamin E (µg/mL) | 39.4 | 40.8 | 39.6 | 31.4 |

**Table 3**

| Results from Comet Data Analysis at One Month | | | | |
|---|---|---|---|---|
| Metabolite | Food A | Food B | Food C | Food D |
| Inherent: | | | | |
| Head DNA (%) | 96.3 | 95.9 | 96.5 | 95.9 |
| Olive Tail Moment | 0.44 | 0.42 | 0.39 | 0.44 |
| Tail Length (µm) | 6.04 | 6.62 . | 6.33 | 6.28 |
| Challenged: | | | | |
| Head DNA (%) | 94.6 | 93.3 | 94.6 | 93.4 |
| Olive Tail Moment | 0.74 | 0.91 | 0.74 | 0.94 |
| Tail Length (µm) | 11.0 | 14.1 | 11.5 | 13.1 |

Referring to Tables 2 and 3, the overall results, particularly the DNA and Tail Length measurements, show that methionine, taurine, vitamin C, and vitamin E in the ranges given by the present invention are useful for enhancing the antioxidant status of an animal.

### Example 2

Example 2 illustrates the effect of the compositions of this invention on enhancing the antioxidant status, improving ORAC, increasing antioxidant amount in blood, and lowering DNA damage in cats.

Forty healthy geriatric cats (twelve years old or older) are fed a control food meeting AAFCO's minimum nutrient level requirements for maintenance for thirty days. After that, the cats are split in four groups, ten cats per group, and each group is fed one of dry foods E, F, G, or H for six months. The contents of foods E, F, G, and H are listed in Table 4.

**Table 4**

| Composition of Foods E, F, G, and H | | | | |
|---|---|---|---|---|
| Food Ingredients (DMB) | Food E | Food F | Food G | Food H |
| Crude Protein (%) | 35.73 | 34.85 | 30.52 | 40.45 |
| Fat (%) | 22.47 | 15.39 | 23.63 | 15.69 |
| Calcium (%) | 0.94 | 1.22 | 0.80 | 1.38 |
| Phosphorus (%) | 0.77 | 1.05 | 0.72 | 1.30 |
| Taurine (ppm) | 2100 | 1800 | 1600 | 2100 |
| Methionine (%) | 1.32 | 1.05 | 0.72 | 0.77 |
| Vitamin E (IU/kg) | 1292 | 390 | 608 | 964 |
| Vitamin C (ppm) | 141 | 12 | 511 | 110 |

Blood samples are drawn after the cats are fed Food E, F, G, or H for one month to determine the amounts of methionine, taurine, vitamin E, and glutathione peroxidase in the blood, the ORAC of the blood, and the ability of the cells to withstand oxidative stress by hydrogen peroxide. Results are presented in Tables 5 and 6.

**Table 5**

| Results from Blood Analysis at One Month | | | | |
|---|---|---|---|---|
| Metabolite | Food E | Food F | Food G | Food H |
| Glutathione Peroxidase (µg/10⁶) | 4.35 | 4.38 | 4.69 | 4.79 |
| Taurine (µmol/L) | 492.9 | 361.3 | 414.8 | 438.7 |
| ORAC (µM) | 3351 | 3499 | 3320 | 3498 |
| Vitamin E (µg/mL) | 23.98 | 20.64 | 21.70 | 22.82 |

**Table 6**

| Results from Comet Data Analysis at One Month | | | | |
|---|---|---|---|---|
| Metabolite | Food E | Food F | Food G | Food H |
| Inherent: | | | | |
| Head DNA (%) | 95.6 | 95.0 | 94.8 | 95.7 |
| Olive Tail Moment | 0.44 | 0.59 | 0.62 | 0.49 |
| Tail Length (µm) | 7.6 | 8.7 | 8.8 | 7.4 |
| Challenged: | | | | |
| Head DNA (%) | 94.2 | 94.1 | 93.7 | 94.2 |
| Olive Tail Moment | 0.72 | 0.75 | 0.79 | 0.74 |
| Tail Length (µm) | 10.0 | 10.9 | 11.4 | 10.7 |

Referring to Tables 5 and 6, the overall results, particularly the DNA and Tail Length measurements, show that methionine, taurine, vitamin C, and vitamin E in the ranges given by the present invention are useful for enhancing the antioxidant status of an animal.

In the specification, there have been disclosed typical preferred embodiments of the invention and, although specific terms are employed, they are used in a generic and descriptive sense only and not for purposes of limitation, the scope of the invention being set forth in the claims. Obviously many modifications and variations of the invention are possible in light of the above teachings. It is therefore to be understood that within the scope of the appended claims this invention may be practiced otherwise than as specifically described.

## Claims

1. A composition for use in enhancing the antioxidant status of an animal comprising from about 0.9 to about 1.5% methionine, from about 1200 to about 5000 ppm taurine, from about 120 to about 450 ppm vitamin C, and from about 700 to about 2000IU/kg vitamin E, all on a dry matter basis.

2. A composition for use in enhancing the antioxidant status of a canine comprising methionine, taurine, vitamin C, and vitamin E, and upon feeding the composition to the canine, the canine's blood comprises from about 300 to about 500µmol/L taurine, optionally from about 300 to about 350µmol/L taurine.

3. A composition for use in enhancing the antioxidant status of a feline comprising methionine, taurine, vitamin C, and vitamin E, and upon feeding the composition to the feline, the feline's blood comprises from about 460 to about 1000µmol/L taurine, optionally from about 460 to about 500µmol/L taurine.

4. The composition of claim 2 or claim 3 wherein the composition comprises from about 0.9 to about 1.5% methionine, and/or from about 1200 to about 5000 ppm taurine, and/or from about 120 to about 450 ppm vitamin C, and/or from about 700 to about 2000IU/kg vitamin E, all on a dry matter basis.

5. The composition of any preceding claim wherein the composition comprises from about 0.9 to about 1.1 % methionine, optionally from about 1.2 to about 1.4% methionine.

6. The composition of any preceding claim wherein the composition comprises from about 1200 to about 1400 ppm taurine, optionally from about 2200 to about 4000 ppm taurine.

7. The composition of any preceding claim wherein the composition comprises from about 130 to about 250 ppm vitamin C.

8. The composition of any preceding claim wherein the composition comprises from about 900 to about 2000IU/lcg vitamin E, optionally from about 1100 to about 2000IU/kg vitamin E.

9. A composition according to any preceding claim wherein the animal is a canine or feline animal.

10. The composition of claim 9 wherein the canine is a dog.

11. The composition of claim 9 wherein the feline is a cat.

12. The composition of claim 10 or claim 11 wherein the canine or feline is a senior or geriatric canine or feline, or at least about 10 years old, or is at least about 12 years old.

13. The composition of any one of claims 9-12 wherein the composition is fed to the canine or feline for at least about three weeks.

14. The composition of any preceding claim which is a food composition.

15. A kit suitable for enhancing the antioxidant status of an animal comprising in separate containers in a single package or in separate containers in a virtual package, as appropriate, a composition comprising from about 0.9 to about 1.5% methionine, from about 1200 to about 5000 ppm taurine, from about 120 to about 450 ppm vitamin C, and from about 700 to about 2000IU/kg vitamin E, or two or more ingredients that, when combined together and, optionally, with additional ingredients which are or are not a part of the kit, yield a composition comprising from about 0.9 to about 1.5% methionine, from about 1200 to about 5000 ppm taurine, from about 120 to about 450 ppm vitamin C, and from about 700 to about 2000IU/kg vitamin E, and one or more of (1) an agent for promoting the health or wellness of the animal, (2) instructions for preparing the composition by combining the ingredients, (3) instructions for feeding the composition to the animal, (4) instructions for administering to the animal an agent for promoting health or wellness in conjunction with feeding the animal the composition, (5) instructions for enhancing the antioxidant status of the animal by feeding the animal the composition, and, (6) instructions for enhancing the antioxidant status of the animal by administering to the animal an agent for promoting health or wellness in conjunction with feeding the animal the composition.
